# EUROPEAN PATENT SPECIFICATION

(11) **EP 2 588 466 B1**
(45) Date of publication and mention of the grant of the patent: **19.03.2014**
(21) Application number: 11723091.2
(22) Date of filing: 07.06.2011
(51) Int. Cl.: C07D 401/10, C07D 401/12, C07D 403/10, A61K 31/496, A61P 25/28, A61P 25/16

(54) **5-Amino-3,6-dihydro-1H-pyrazin-2-one derivatives useful as inhibitors of beta-secretase (BACE)**
5-Amino-3,6-dihydro-1H-pyrazin-2-on-Derivate als Hemmer von Beta-Sekretase (BACE)
Dérivés de la 5-amino-3,6-dihydro-1H-pyrazin-2-one utiles en tant qu'inhibiteurs de la bêta-sécrétase (BACE)

(30) Priority: 09.06.2010 EP 10165336
(43) Date of publication of application: 08.05.2013
(73) Proprietor: Janssen Pharmaceutica, N.V., 2340 Beerse (BE)
(72) Inventor: TRABANCO-SUÁREZ, Andrés, Avelino, E-45007 Toledo (ES); TRESADERN, Gary, John, E-45007 Toledo (ES); MACDONALD, Gregor, James, B-2340 Beerse (BE); VEGA RAMIRO, Juan, Antonio, E-45007 Toledo (ES)
(74) Representative: Quaghebeur, Luc
(86) International application number: PCT/EP2011/059330
(87) International publication number: WO 2011/154374

(56) References cited:
- EP-A1- 2 147 914
- WO-A1-2007/005404
- WO-A1-2007/114771
- WO-A1-2009/134617
- US-A1- 2005 282 825
- US-A1- 2007 225 372
- SILVESTRI R: "Boom in the development of non-peptidic beta-secretase (BACE1) inhibitors for the treatment of Alzheimer's disease", MEDICINAL RESEARCH REVIEWS, NEW YORK, NY, US LNKD- DOI:10.1002/MED.20132, vol. 29, no. 2, 1 March 2009 (2009-03-01), pages 295-338, XP002536842, ISSN: 0198-6325

## Description

### FIELD OF THE INVENTION

The present invention relates to novel 5-amino-3,6-dihydro-1*H*-pyrazin-2-one derivatives as inhibitors of beta-secretase, also known as beta-site amyloid cleaving enzyme, BACE, BACE1, Asp2, or memapsin2. The invention is also directed to pharmaceutical compositions comprising such compounds, to processes for preparing such compounds and compositions, and to the use of such compounds and compositions for the prevention and treatment of disorders in which beta-secretase is involved, such as Alzheimer's disease (AD), mild cognitive impairment, senility, dementia, dementia with Lewy bodies, Down's syndrome, dementia associated with stroke, dementia associated with Parkinson's disease or dementia associated with beta-amyloid.

### BACKGROUND OF THE INVENTION

Alzheimer's Disease (AD) is a neurodegenerative disease associated with aging. AD patients suffer from cognition deficits and memory loss as well as behavioral problems such as anxiety. Over 90% of those afflicted with AD have a sporadic form of the disorder while less than 10% of the cases are familial or hereditary. In the United States, about 1 in 10 people at age 65 have AD while at age 85, 1 out of every two individuals are affected with AD. The average life expectancy from the initial diagnosis is 7-10 years, and AD patients require extensive care either in an assisted living facility which is very costly or by family members. With the increasing number of elderly in the population, AD is a growing medical concern. Currently available therapies for AD merely treat the symptoms of the disease and include acetylcholinesterase inhibitors to improve cognitive properties as well as anxiolytics and antipsychotics to control the behavioral problems associated with this ailment.

The hallmark pathological features in the brain of AD patients are neurofibillary tangles which are generated by hyperphosphorylation of tau protein and amyloid plaques which form by aggregation of beta-amyloid 1-42 (Abeta 1-42) peptide. Abeta 1-42 forms oligomers and then fibrils, and ultimately amyloid plaques. The oligomers and fibrils are believed to be especially neurotoxic and may cause most of the neurological damage associated with AD. Agents that prevent the formation of Abeta 1-42 have the potential to be disease-modifying agents for the treatment of AD. Abeta 1-42 is generated from the amyloid precursor protein (APP), comprised of 770 amino acids. The N-terminus of Abeta 1-42 is cleaved by beta-secretase (BACE), and then gamma-secretase cleaves the C-terminal end. In addition to Abeta 1-42, gamma-secretase also liberates Abeta 1-40 which is the predominant cleavage product as well as Abeta 1-38 and Abeta 1-43. These Abeta forms can also aggregate to form oligomers and fibrils. Thus, inhibitors of BACE would be expected to prevent the formation of Abeta 1-42 as well as Abeta 1-40, Abeta 1-38 and Abeta 1-43 and would be potential therapeutic agents in the treatment of AD.

### SUMMARY OF THE INVENTION

The present invention is directed to 5-amino-3,6-dihydro-1*H* pyrazin-2-ones of Formula (I) 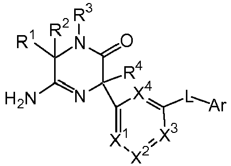 and the stereoisomeric forms thereof, wherein
R¹, R² are hydrogen;
R³, R⁴ are independently methyl or ethyl;
X¹ and X³ are CH or CF;
X² and X⁴ are CH;
L is a bond or -N(R⁶)CO- wherein R⁶ is hydrogen;
Ar is heteroaryl;
heteroaryl is selected from the group consisting of pyridyl, pyrimidinyl and pyrazyl, each optionally substituted with chloro, cyano, methyl, methoxy or trifluoromethyl.

Illustrative of the invention is a pharmaceutical composition comprising a pharmaceutically acceptable carrier and any of the compounds described above. An illustration of the invention is a pharmaceutical composition made by mixing any of the compounds described above and a pharmaceutically acceptable carrier. Illustrating the invention is a process for making a pharmaceutical composition comprising mixing any of the compounds described above and a pharmaceutically acceptable carrier.

An example of the invention is any of the compounds described above for use in treating: (a) Alzheimer's Disease, (b) mild cognitive impairment, (c) senility, (d) dementia, (e) dementia with Lewy bodies, (f) Down's syndrome, (g) dementia associated with stroke, (h) dementia associated with Parkinson's disease and (i) dementia associated with beta-amyloid, in a subject in need thereof.

### DETAILED DESCRIPTION OF THE INVENTION

The present invention is directed to compounds of formula (I) as defined hereinbefore, and pharmaceutically acceptable salts thereof. The compounds of formula (I) are inhibitors of the beta-secretase enzyme (also known as beta-site cleaving enzyme, BACE, BACE1 , Asp2 or memapsin 2), and are useful in the treatment of Alzheimer's disease, mild cognitive impairment, senility, dementia, dementia associated with stroke, dementia with Lewy bodies, Down's syndrome, dementia associated with Parkinson's disease and dementia associated with beta-amyloid, preferably Alzheimer's disease, mild cognitive impairment or dementia, more preferably Alzheimer's disease.

In an embodiment of the present invention.

R¹, R² are hydrogen; R³, R⁴ are methyl; X¹, X², X³, X⁴ are CH; L is -N(R⁶)CO- wherein R⁶ is hydrogen; Ar is heteroaryl; heteroaryl is pyridyl substituted with chloro, cyano, methoxy or trifluoromethyl, pyrimidinyl, or pyrazyl substituted with methyl.

### DEFINITIONS

"Halo" shall denote fluoro, chloro and bromo; "C₁₋₃alkyl" shall denote a straight or branched saturated alkyl group having 1, 2 or 3 carbon atoms, e.g. methyl, ethyl, 1-propyl and 2-propyl; "C₁₋₃alkyloxy" shall denote an ether radical wherein C₁₋₃alkyl is as defined before; "mono- andpolyhaloC₁₋₃alkyl" shall denote C₁₋₃alkyl as defined before, substituted with 1, 2, 3 or where possible with more halo atoms as denied before; "mono- and polyhaloC₁₋₃alkyloxy" shall denote an ether radical wherein monoand polyhaloC₁₋₃alkyl is as defined before; "C₃₋₆cycloalkyl" shall denote cyclopropyl, cyclobutyl, cyclopentyl and cyclohexyl; "C₃₋₆cycloalkanediyl" shall denote a bivalent radical such as cyclopropanediyl, cyclobutanediyl, cyclopentanediyl and cyclohexanediyl.

The term "subject" as used herein, refers to an animal, preferably a mammal, most preferably a human, who is or has been the object of treatment, observation or experiment.

The term "therapeutically effective amount" as used herein, means that amount of active compound or pharmaceutical agent that elicits the biological or medicinal response in a tissue system, animal or human that is being sought by a researcher, veterinarian, medical doctor or other clinician, which includes alleviation of the symptoms of the disease or disorder being treated.

As used herein, the term "composition" is intended to encompass a product comprising the specified ingredients in the specified amounts, as well as any product which results, directly or indirectly, from combinations of the specified ingredients in the specified amounts.

It will be appreciated that some of the compounds according to formula (I) and the addition salts, hydrates and solvates thereof may contain one or more centers of chirality and exist as stereoisomeric forms.

Hereinbefore and hereinafter, the term "compound of formula (I)" is meant to include the addition salts, the solvates and the stereoisomers thereof.

The terms "stereoisomers" or "stereochemically isomeric forms" hereinbefore or hereinafter are used interchangeably.

The invention includes all stereoisomers of the compound of Formula (I) either as a pure stereoisomer or as a mixture of two or more stereoisomers.

Enantiomers are stereoisomers that are non-superimposable mirror images of each other. A 1:1 mixture of a pair of enantiomers is a racemate or racemic mixture. Diastereomers (or diastereoisomers) are stereoisomers that are not enantiomers, i.e. they are not related as mirror images. If a compound contains a double bond, the substituents may be in the E or the Z configuration. If a compound contains a disubstituted cycloalkyl group, the substituents may be in the cis or trans configuration. Therefore, the invention includes enantiomers, diastereomers, racemates, E isomers, Z isomers, cis isomers, trans isomers and mixtures thereof.

The absolute configuration is specified according to the Cahn-Ingold-Prelog system. The configuration at an asymmetric atom is specified by either R or S. Resolved compounds whose absolute configuration is not known can be designated by (+) or (-) depending on the direction in which they rotate plane polarized light.

When a specific stereoisomer is identified, this means that said stereoisomer is substantially free, i.e. associated with less than 50%, preferably less than 20%, more preferably less than 10%, even more preferably less than 5%, in particular less than 2% and most preferably less than 1%, of the other isomers. Thus, when a compound of formula (I) is for instance specified as (R), this means that the compound is substantially free of the (S) isomer; when a compound of formula (I) is for instance specified as E, this means that the compound is substantially free of the Z isomer; when a compound of formula (I) is for instance specified as cis, this means that the compound is substantially free of the trans isomer.

Furthermore, some of the crystalline forms for the compounds of the present invention may exist as polymorphs and as such are intended to be included in the present invention. In addition, some of the compounds of the present invention may form solvates with water (i.e., hydrates) or common organic solvents, and such solvates are also intended to be encompassed within the scope of this invention.

For use in medicine, the salts of the compounds of this invention refer to nontoxic "pharmaceutically acceptable salts". Other salts may, however, be useful in the preparation of compounds according to this invention or of their pharmaceutically acceptable salts. Suitable pharmaceutically acceptable salts of the compounds include acid addition salts which may, for example, be formed by mixing a solution of the compound with a solution of a pharmaceutically acceptable acid such as hydrochloric acid, sulfuric acid, fumaric acid, maleic acid, succinic acid, acetic acid, benzoic acid, citric acid, tartaric acid, carbonic acid or phosphoric acid. Furthermore, where the compounds of the invention carry an acidic moiety, suitable pharmaceutically acceptable salts thereof may include alkali metal salts, e.g., sodium or potassium salts; alkaline earth metal salts, e.g., calcium or magnesium salts; and salts formed with suitable organic ligands, e.g., quaternary ammonium salts.

Representative acids which may be used in the preparation of pharmaceutically acceptable salts include, but are not limited to, the following: acetic acid, 2,2-dichloroactic acid, acylated amino acids, adipic acid, alginic acid, ascorbic acid, L-aspartic acid, benzenesulfonic acid, benzoic acid, 4- acetamidobenzoic acid, (+)-camphoric acid, camphorsulfonic acid, capric acid, caproic acid, caprylic acid, cinnamic acid, citric acid, cyclamic acid, ethane-1,2-disulfonic acid, ethanesulfonic acid, 2-hydroxyethanesulfonic acid, formic acid, fumaric acid, galactaric acid, gentisic acid, glucoheptonic acid, D-gluconic acid, D-glucoronic acid, L-glutamic acid, beta-oxo-glutaric acid, glycolic acid, hippuric acid, hydrobromic acid, hydrochloric acid, (+)-L-lactic acid, (±)-DL-lactic acid, lactobionic acid, maleic acid, (-)-L-malic acid, malonic acid, (±)-DL-mandelic acid, methanesulfonic acid, naphthalene-2-sulfonic acid, naphthalene-1,5- disulfonic acid, 1-hydroxy-2-naphthoic acid, nicotinic acid, nitric acid, oleic acid, orotic acid, oxalic acid, palmitic acid, pamoic acid, phosphoric acid, L- pyroglutamic acid, salicylic acid, 4-amino-salicylic acid, sebacic acid, stearic acid, succinic acid, sulfuric acid, tannic acid, (+)-L-tartaric acid, thiocyanic acid, p-toluenesulfonic acid, trifluoromethylsulfonic acid, and undecylenic acid. Representative bases which may be used in the preparation of pharmaceutically acceptable salts include, but are not limited to, the following: ammonia, L-arginine, benethamine, benzathine, calcium hydroxide, choline, dimethylethanolamine, diethanolamine, diethylamine, 2-(diethylamino)-ethanol, ethanolamine, ethylene-diamine, *N*-methyl-glucamine, hydrabamine, 1*H-*imidazole, L-lysine, magnesium hydroxide, 4-(2-hydroxyethyl)-morpholine, piperazine, potassium hydroxide, 1-(2-hydroxyethyl)-pyrrolidine, secondary amine, sodium hydroxide, triethanolamine, tromethamine and zinc hydroxide.

The chemical names of the compounds of the present invention were generated according to the nomenclature rules agreed upon by the Chemical Abstracts Service.

Some of the compounds according to formula (I) may also exist in their tautomeric form. Such forms although not explicitly indicated in the above formula are intended to be included within the scope of the present invention.

### PREPARATION OF THE COMPOUNDS

### Experimental procedure 1

The final compounds according to Formula (I), can be prepared by reacting an intermediate compound of Formula (II) with an appropriate source of ammonia such as, for example, ammonium chloride or aqueous ammonia, according to reaction scheme (1), a reaction that is performed in a suitable reaction-inert solvent, such as, for example, water or methanol, under thermal conditions such as, for example, heating the reaction mixture at 60°C, for example for 6 hours. In reaction scheme (1), all variables are defined as in Formula (I).

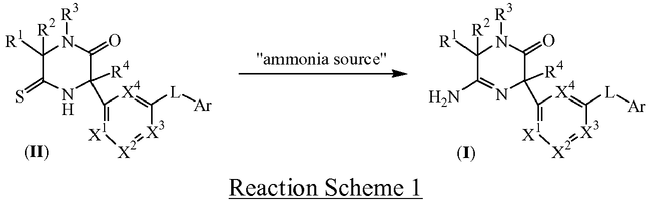

### Experimental procedure 2

The final compounds according to Formula (I-a) wherein L is -N(R⁶)CO-, can be prepared by reacting an intermediate compound of Formula (III-a) with a compound of Formula (IV) according to reaction scheme (2), a reaction that is performed in a suitable reaction-inert solvent, such as, for example, *N,N-*dimethylformamide, in the presence of a suitable base, such as, for example, K₃PO₄, a copper catalyst such as, for example, CuI and a diamine such as for example (1*R*,2*R*)-(-)-1,2-diaminocyclohexane, under thermal conditions such as, for example, heating the reaction mixture at 180°C, for example for 140 minutes under microwave irradiation. In reaction scheme (2), all variables are defined as in Formula (I) and W is halo.

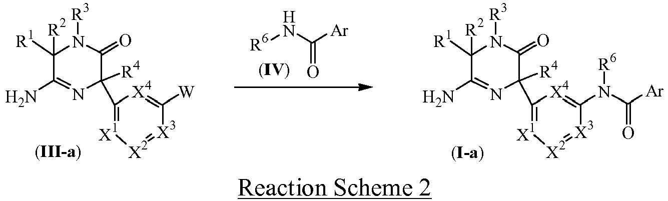

### Experimental procedure 3

Additionally, the final compounds according to Formula (I-a), can be prepared by reacting an intermediate compound of Formula (III-b) with a compound of Formula (V) according to reaction scheme (3), a reaction that is performed in a suitable reaction-inert solvent, such as, for example, dichloromethane, in the presence of a suitable base, such as, for example, triethylamine, in the presence of a condensation agent such as for example *O*-(7-azabenzotriazol-1-yl)-*N,N,N',N'*-tetramethyluronium hexafluorophosphate [HATU, CAS 148893-10-1], under thermal conditions such as, for example, heating the reaction mixture at 25°C, for example for 2 hours. In reaction scheme (3), all variables are defined as in Formula (I).

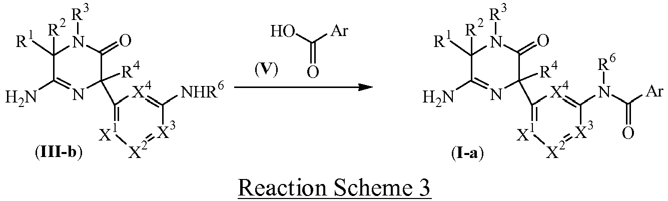

### Experimental procedure 4

Additionally, the final compounds according to Formula (I-a), can be prepared by reacting an intermediate compound of Formula (III-b) with a compound of Formula (VI) according to reaction scheme (4), a reaction that is performed in a suitable reaction-inert solvent, such as, for example, dichloromethane, in the presence of a suitable base, such as, for example, pyridine, under thermal conditions such as, for example, heating the reaction mixture at 25 °C, for example for 2 hours. In reaction scheme (4), all variables are defined as in Formula (I) and Y is halo.

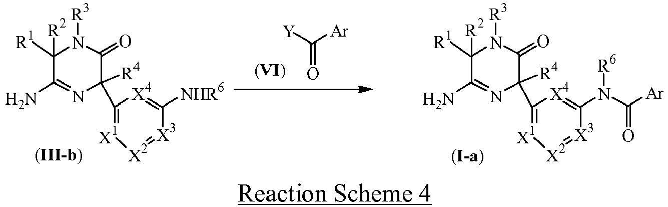

### Experimental procedure 5

The final compounds according to Formula (I-b) wherein L is a bond, can be prepared by reacting an intermediate compound of Formula (III-a) with a compound of Formula (VII) according to reaction scheme (5), a reaction that is performed in a suitable reaction-inert solvent, such as, for example, ethanol or mixtures of inert solvents such as, for example, 1,2-dimethoxyethane/water/ethanol, in the presence of a suitable base, such as, for example, aqueous K₃PO₄ or Cs₂CO₃, a Pd-complex catalyst such as, for example, [1,1'-bis(diphenylphosphino)ferrocene]dichloropalladium(II) [CAS 72287-26-4] or trans-(bisdicyclohexylamine)palladium diacetate [DAPCy, CAS 628339-96-8] under thermal conditions such as, for example, heating the reaction mixture at 80 °C, for example for 48 hours or for example , heating the reaction mixture at 130 °C, for example for 10 minutes under microwave irradiation. In reaction scheme (5), all variables are defined as in Formula (I) and W is halo. R⁷ and R⁸ may be hydrogen or alkyl, or may be taken together to form for example a bivalent radical of formula -CH₂CH₂-, -CH₂CH₂CH₂-, or -C(CH₃)₂C(CH₃)₂-.

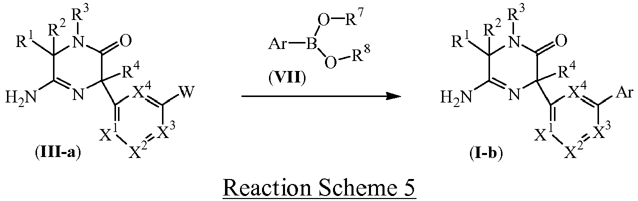

A number of intermediates and starting materials in the foregoing preparations are known compounds which may be prepared according to art-known methodologies of preparing said or similar compounds and some intermediates are new. A number of such preparation methods will be described hereinafter in more detail.

### Experimental procedure 6

The intermediates according to Formula (II) can be prepared by reacting an intermediate compound of Formula (VIII) with a suitable sulphur donating reagent for the synthesis of thioamides such as, for example, phosphorous pentasulfide or 2,4-bis-(4-methoxyphenyl)-1,3-dithia-2,4-diphosphetane 2,4-disulfide [Lawesson's reagent, CAS 19172-47-5] according to reaction scheme (6), a reaction that is performed in a reaction inert solvent, such as for example, tetrahydrofuran or toluene, in the presence of a suitable base such as, for example, pyridine, under thermal conditions such as, for example, heating the reaction mixture at 90 °C, for example for 18 hours. In reaction scheme (6), all variables are defined as in Formula (I).

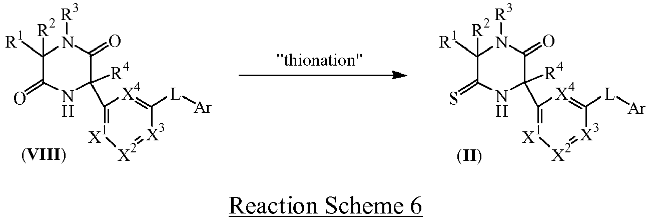

### Experimental procedure 7

The intermediates according to Formula (VIII) wherein L is a bond, can be prepared by reacting an intermediate compound of Formula (IX-a) with a compound of Formula (VII) according to reaction scheme (7), a reaction that is performed in a suitable mixture of inert solvents such as, for example, 1,4-dioxane/water , in the presence of a suitable base, such as, for example, aqueous Na₂CO₃, a Pd-complex catalyst such as, for example, tetrakis-(triphenylphosphine)palladium (0) [CAS 14221-01-3] under thermal conditions such as, for example, heating the reaction mixture at 80 °C, for example for 20 hours or for example , heating the reaction mixture at 150 °C, for example for 15 minutes under microwave irradiation. In reaction scheme (7), all variables are defined as in Formula (I) and W is halo. R⁷ and R⁸ may be hydrogen or alkyl, or may be taken together to form for example a bivalent radical of formula -CH₂CH₂-, -CH₂CH₂CH₂-, or -C(CH₃)₂C(CH₃)₂-.

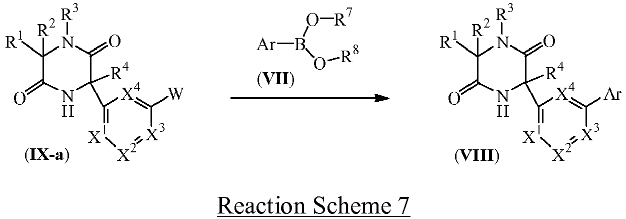

### Experimental procedure 8

The intermediate compounds of Formula (III-a), (III-b) and (III-c) can generally be prepared following the reaction steps shown in the reaction schemes (8) and (9) below.

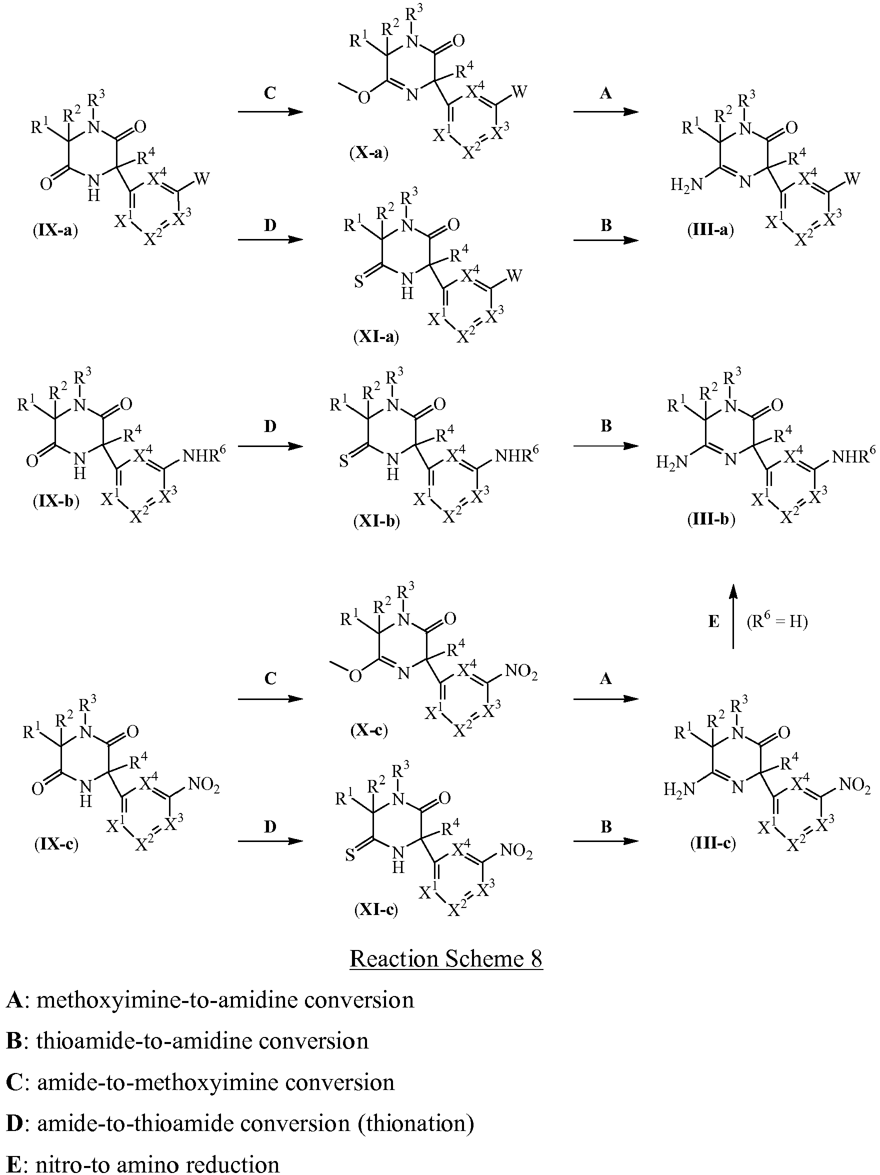

Intermediate compounds of Formula (III-a), (III-b) and (III-c) in the above reaction scheme (8) can be prepared from the corresponding intermediate compounds of Formula (XI-a), (XI-b) and (XI-c) following art-known thioamide-to-amidine conversion procedures (reaction step B) or alternatively, for intermediate compounds of Formula (III-a) and (III-c), from the corresponding intermediate compounds of Formula (X-a) and (X-c) following art-known methoxyimine-to-amidine conversion procedures (reaction step A). Said conversions may conveniently be conducted by treatment of the corresponding intermediate compounds of Formula (XI-a), (XI-b) and (XI-c) or (X-a) and (X-c) with an ammonia source such as, for example, ammonium chloride or aqueous ammonia, in a suitable reaction-inert solvent such as, for example, water or methanol and the like, under thermal conditions such as, for example, heating the reaction mixture at 70 °C to 85 °C, for example, for 6 hours to 18 hours.

Additionally intermediate compounds of Formula (III-b) in the above reaction scheme (8), wherein R⁶ = H, can be prepared from the corresponding intermediate compounds of Formula (III-c) following art-known nitro-to-amino reduction procedures (reaction step E). Said reduction may conveniently be conducted following art-known catalytic hydrogenation procedures. For example, said reduction may be carried out by stirring the reactants under a hydrogen atmosphere and in the presence of an appropriate catalyst such as, for example, palladium-on-charcoal, platinum-on-charcoal, Raney-nickel and the like catalysts. Suitable solvents are, for example, water, alkanols, e.g. methanol, ethanol and the like, esters, e.g. ethyl acetate and the like. In order to enhance the rate of said reduction reaction it may be advantageous to elevate the temperature and/or the pressure of the reaction mixture. Undesired further hydrogenation of certain functional groups in the reactants and the reaction products may be prevented by the addition of a catalyst poison such as, for example, thiophene and the like, to the reaction mixture.

Intermediate compounds of Formula (X-a) and (X-c) in the above reaction scheme (8) can be prepared from the corresponding intermediate compounds of Formula (IX-a) and (IX-c) following art-known amide-to-methoxyimine conversion procedures (reaction step C) Said conversion may conveniently be conducted by treatment of the corresponding intermediate compounds of Formula (IX-a) and (IX-c) with a methylating agent such as, for example, trimethyloxonium tetrafluoroborate, in a suitable reaction-inert solvent such as, for example, dichloromethane, at a moderately high temperature such as, for example, 25 °C, for example for 60 hours.

The thioamide derivatives of Formula (XI-a), (XI-b) and (XI-c) in the above reaction scheme (8) can be prepared from amide derivatives of Formula (IX-a), (IX-b) and (IX-c) following art-known thionation procedures (reaction step D). Said conversion may conveniently be conducted by treatment of the said amides with a thionation agent such as, for example, phosphorous pentasulfide or 2,4-bis-(4-methoxy-phenyl)-1,3-dithia-2,4-diphosphetane 2,4-disulfide [Lawesson's reagent, CAS 19172-47-5], in the presence of a suitable base, such as, for example, pyridine, in a reaction inert solvent such as, for example, tetrahydrofuran or toluene, under thermal conditions such as, for example, heating the reaction mixture at 90 °C, for example for 18 hours.

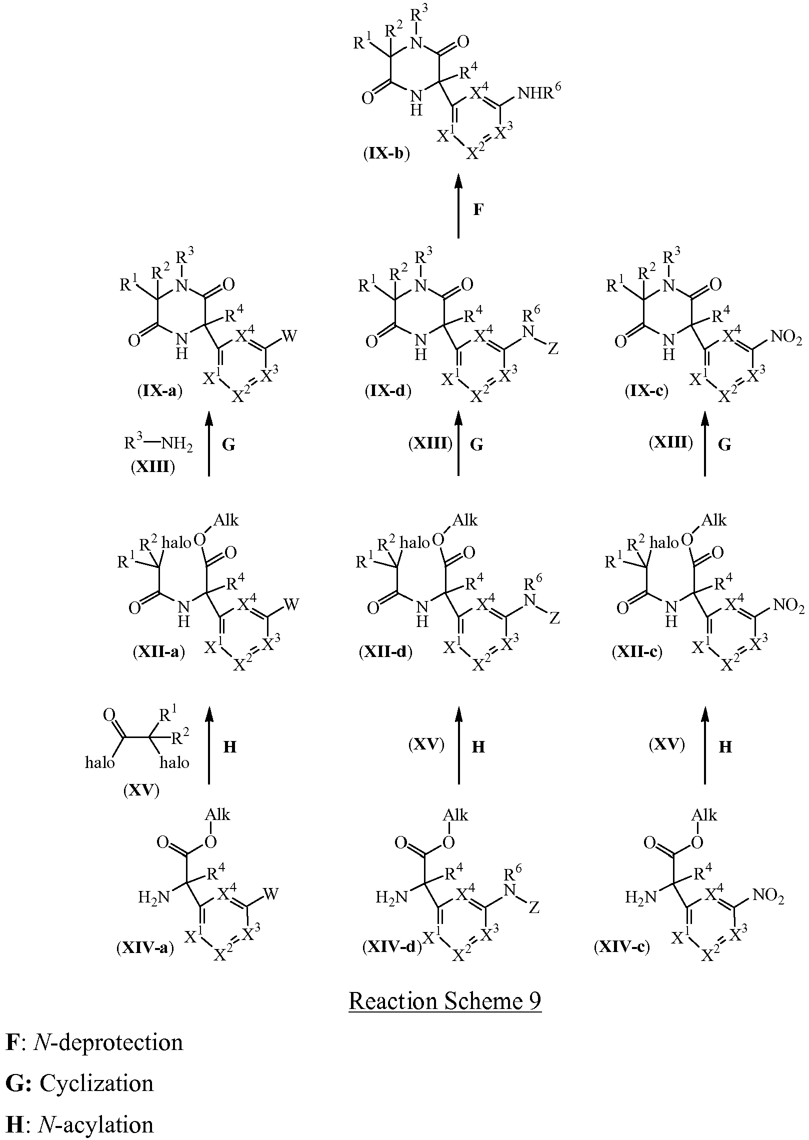

The intermediates according to Formula (IX-b) in the above reaction scheme (9) can be prepared by reacting an intermediate compound of Formula (IX-d), wherein Z is a protecting group of amines such as, for example, the *p*-methoxybenzyl group, following art-known *N*-deprotection procedures of amines (reaction step F). Said *N*-deprotection may conveniently be conducted by treatment of the corresponding intermediate compounds of Formula (IX-d) with a suitable deprotecting agent of the amine function such as, for example, ammonium cerium (IV) nitrate, in a mixture of inert solvents such as, for example, acetonitrile/water, at a moderately high temperature such as, for example, 25 °C, for example for 4 hours.

The intermediates according to Formula (IX-a), (IX-c) and (IX-d) in the above reaction scheme (9) can be prepared by reacting an intermediate compound of Formula (XII-a), (XII-c) and (XII-d) following art-known cyclization procedures (reaction step G). Said cyclization may conveniently be conducted by treatment of the corresponding intermediate compounds of Formula (XII-a), (XII-c) and (XII-d) with an intermediate compound of Formula (XIII) a reaction that is performed in a suitable reaction-inert solvent, such as, for example, ethanol, under thermal conditions such as, for example, heating the reaction mixture at 70 °C, for example for 3 hours. In reaction scheme (9), all variables are defined as in Formula (I), halo is chloro or bromo and Alk is C₁₋₃alkyl.

The intermediates according to Formula (XII-a), (XII-c) and (XII-d) in the above reaction scheme (9) can be prepared by reacting an intermediate compound of Formula (XIV-a), (XIV-c) and (XIV-d) following art-known *N*-acylation procedures (reaction step H). Said *N*-acylation may conveniently be conducted by treatment of the corresponding intermediate compounds of Formula (XIV-a), (XIV-c) and (XIV-d) with an intermediate compound of Formula (XV) a reaction that is performed in a suitable reaction-inert solvent, such as, for example, dichloromethane, in the presence of a suitable base, such as, for example, triethylamine, at low temperature such as, for example, 0 °C, for example for 1 hour. In reaction scheme (9), all variables are defined as in Formula (I), halo is chloro or bromo and Alk is C₁₋₃alkyl.

The intermediates compounds of Formula (XIV-a), (XIV-c) and (XIV-d), wherein Z is a suitable *N*-protecting group such as, for example the *p*-methoxybenzyl group, can generally be prepared following art-known Strecker type procedures.

### PHARMACEUTICAL COMPOSITIONS

The present invention also provides compositions for preventing or treating diseases in which inhibition of beta-secretase is beneficial, such as Alzheimer's disease (AD), mild cognitive impairment, senility, dementia, dementia with Lewy bodies, Down's syndrome, dementia associated with stroke, dementia associated with Parkinson's disease and dementia associated with beta-amyloid. Said compositions comprising a therapeutically effective amount of a compound according to formula (I) and a pharmaceutically acceptable carrier or diluent.

While it is possible for the active ingredient to be administered alone, it is preferable to present it as a pharmaceutical composition. Accordingly, the present invention further provides a pharmaceutical composition comprising a compound according to the present invention, together with a pharmaceutically acceptable carrier or diluent. The carrier or diluent must be "acceptable" in the sense of being compatible with the other ingredients of the composition and not deleterious to the recipients thereof.

The pharmaceutical compositions of this invention may be prepared by any methods well known in the art of pharmacy. A therapeutically effective amount of the particular compound, in base form or addition salt form, as the active ingredient is combined in intimate admixture with a pharmaceutically acceptable carrier, which may take a wide variety of forms depending on the form of preparation desired for administration. These pharmaceutical compositions are desirably in unitary dosage form suitable, preferably, for systemic administration such as oral, percutaneous or parenteral administration; or topical administration such as via inhalation, a nose spray, eye drops or via a cream, gel, shampoo or the like. For example, in preparing the compositions in oral dosage form, any of the usual pharmaceutical media may be employed, such as, for example, water, glycols, oils, alcohols and the like in the case of oral liquid preparations such as suspensions, syrups, elixirs and solutions: or solid carriers such as starches, sugars, kaolin, lubricants, binders, disintegrating agents and the like in the case of powders, pills, capsules and tablets. Because of their ease in administration, tablets and capsules represent the most advantageous oral dosage unit form, in which case solid pharmaceutical carriers are obviously employed. For parenteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed. In the compositions suitable for percutaneous administration, the carrier optionally comprises a penetration enhancing agent and/or a suitable wettable agent, optionally combined with suitable additives of any nature in minor proportions, which additives do not cause any significant deleterious effects on the skin. Said additives may facilitate the administration to the skin and/or may be helpful for preparing the desired compositions. These compositions may be administered in various ways, e.g., as a transdermal patch, as a spot-on or as an ointment.

It is especially advantageous to formulate the aforementioned pharmaceutical compositions in dosage unit form for ease of administration and uniformity of dosage. Dosage unit form as used in the specification and claims herein refers to physically discrete units suitable as unitary dosages, each unit containing a predetermined quantity of active ingredient calculated to produce the desired therapeutic effect in association with the required pharmaceutical carrier. Examples of such dosage unit forms are tablets (including scored or coated tablets), capsules, pills, powder packets, wafers, injectable solutions or suspensions, teaspoonfuls, tablespoonfuls and the like, and segregated multiples thereof.

The exact dosage and frequency of administration depends on the particular compound of formula (I) used, the particular condition being treated, the severity of the condition being treated, the age, weight, sex, extent of disorder and general physical condition of the particular patient as well as other medication the individual may be taking, as is well known to those skilled in the art. Furthermore, it is evident that said effective daily amount may be lowered or increased depending on the response of the treated subject and/or depending on the evaluation of the physician prescribing the compounds of the instant invention.

Depending on the mode of administration, the pharmaceutical composition will comprise from 0.05 to 99 % by weight, preferably from 0.1 to 70 % by weight, more preferably from 0.1 to 50 % by weight of the active ingredient, and, from 1 to 99.95 % by weight, preferably from 30 to 99.9 % by weight, more preferably from 50 to 99.9 % by weight of a pharmaceutically acceptable carrier, all percentages being based on the total weight of the composition.

The present compounds can be used for systemic administration such as oral, percutaneous or parenteral administration; or topical administration such as via inhalation, a nose spray, eye drops or via a cream, gel, shampoo or the like. The compounds are preferably orally administered. The exact dosage and frequency of administration depends on the particular compound according to formula (I) used, the particular condition being treated, the severity of the condition being treated, the age, weight, sex, extent of disorder and general physical condition of the particular patient as well as other medication the individual may be taking, as is well known to those skilled in the art. Furthermore, it is evident that said effective daily amount may be lowered or increased depending on the response of the treated subject and/or depending on the evaluation of the physician prescribing the compounds of the instant invention.

The amount of a compound of Formula (I) that can be combined with a carrier material to produce a single dosage form will vary depending upon the disease treated, the mammalian species, and the particular mode of administration. However, as a general guide, suitable unit doses for the compounds of the present invention can, for example, preferably contain between 0.1 mg to about 1000 mg of the active compound. A preferred unit dose is between 1 mg to about 500 mg. A more preferred unit dose is between 1 mg to about 300mg. Even more preferred unit dose is between 1 mg to about 100 mg. Such unit doses can be administered more than once a day, for example, 2, 3, 4, 5 or 6 times a day, but preferably 1 or 2 times per day, so that the total dosage for a 70 kg adult is in the range of 0.001 to about 15 mg per kg weight of subject per administration. A preferred dosage is 0.01 to about 1.5 mg per kg weight of subject per administration, and such therapy can extend for a number of weeks or months, and in some cases, years. It will be understood, however, that the specific dose level for any particular patient will depend on a variety of factors including the activity of the specific compound employed; the age, body weight, general health, sex and diet of the individual being treated; the time and route of administration; the rate of excretion; other drugs that have previously been administered; and the severity of the particular disease undergoing therapy, as is well understood by those of skill in the area.

A typical dosage can be one 1 mg to about 100 mg tablet or 1 mg to about 300 mg taken once a day, or, multiple times per day, or one time-release capsule or tablet taken once a day and containing a proportionally higher content of active ingredient. The time-release effect can be obtained by capsule materials that dissolve at different pH values, by capsules that release slowly by osmotic pressure, or by any other known means of controlled release.

It can be necessary to use dosages outside these ranges in some cases as will be apparent to those skilled in the art. Further, it is noted that the clinician or treating physician will know how and when to start, interrupt, adjust, or terminate therapy in conjunction with individual patient response.

For the compositions, methods and kits provided above, one of skill in the art will understand that preferred compounds for use in each are those compounds that are noted as preferred above. Still further preferred compounds for the compositions, methods and kits are those compounds provided in the non-limiting Examples below.

### EXPERIMENTAL PART

Hereinafter, the term 'm.p." means melting point, 'THF' means tetrahydrofuran, 'DMF' means dimethylformamide, 'DCM' means dichloromethane, 'AcOEt' means ethylacetate, "AcOH" means acetic acid, "MeOH" means methanol, "rac" means racemic.

### A. Preparation of the intermediates

### Example A1

### Preparation of intermediate 1: rac-2-amino-2-(3-bromo-phenyl)-propionitrile

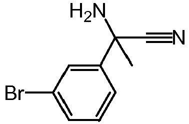

Trimethylsilylcyanide (25.+-2 mL, 201 mmol) was added to a stirred solution of 3-bromo-acetophenone (25 g, 125.6 mmol) and NH₄Cl (13.4 g, 251.2 mmol) in NH₃/MeOH (500 mL). The mixture was stirred at room temperature for 4 days. Then the solvent was evaporated *in vacuo* and the residue was taken up in AcOEt. The solid was filtered off and the solvent was evaporated *in vacuo* to yield **intermediate 1** (26 g, 92% yield) that was used in the next step without further purification.

### Example A2

### Preparation of intermediate 2: rac-2-amino-2-(3-bromo-phenyl)-propionic acid

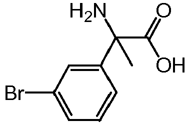

**Intermediate 1** (26 g, 115.5 mmol) was dissolved in 6N HCl (139 mL) and the mixture was refluxed for 18 hours. After cooling to room temperature, the solvents were evaporated *in vacuo* to yield **intermediate 2** (24 g, 85% yield) that was used in the next step without further purification.

### Example A3

### Preparation of intermediate 3: rac-2-amino-2-(3-bromo-phenyl)-propionic acid methyl ester

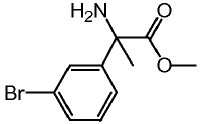

Thionyl chloride (8.97 mL, 122.9 mmol) was added dropwise to a stirred solution of **intermediate 2** (10 g, 41 mmol) in MeOH (125 mL) at 0 °C. Then, the mixture was refluxed for 18 hours. The solvents were evaporated *in vacuo* and the residue was partitioned between Na₂CO₃ (aqueous sat. soltn.) and DCM. The organic layer was separated, dried (Na₂SO₄), filtered and the solvents evaporated *in vacuo.* The crude product was purified by flash column chromatography (silica gel; AcOEt in DCM 0/100 to 30/70). The desired fractions were collected and concentrated *in vacuo* to yield **intermediate 3**(4.1 g, 39% yield) as a colourless oil.

### Example A4

### Preparation of intermediate 4: 2-(3-bromo-phenyl)-2-(2-chloro-acetylamino)-propionic acid methyl ester

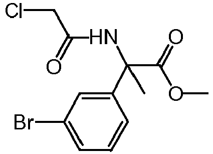

Chloroacetyl chloride (0.34 mL, 4.26 mmol) was added dropwise to a stirred solution of **intermediate 3** (1 g, 3.87 mmol) and Et₃N (0.74 mL, 5.81 mmol) in DCM (35 mL) under nitrogen at 0 °C. The mixture was stirred at 0 °C for 1 hour. Then the mixture was diluted with water and extracted with DCM. The organic layer was separated, dried (Na₂SO₄), filtered and the solvents evaporated *in vacuo* to yield **intermediate 4** (1.3 g, 89% yield) that was used in the next step without further purification.

### Example A5

### Preparation of intermediate 5: rac-3-(3-bromo-phenyl)-1,3-dimethyl-piperazine-2,5-dione

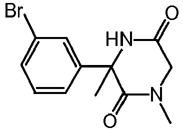

Methylamine 33% in EtOH (5.36 mL, 43.04 mmol) was added to a stirred solution of **intermediate 4** (2.4 g, 7.17 mmol) in EtOH (53 mL) in a sealed tube at room temperature. Then, the mixture was stirred at 70 °C for 3 hours. The solvent was evaporated *in vacuo* to yield **intermediate 5** (1.95 g, 88% yield) that was used in the next step without further purification.

### Example A6

### Preparation of intermediate 6: rac-1,3-dimethyl-3-(3-pyrimidin-5-yl-phenyl)-piperazine-2,5-dione

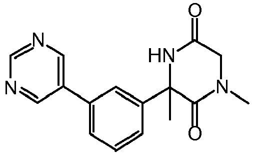

Tetrakis(triphenylphosphine)palladium (0) (0.023 g, 0.020 mmol) was added to a stirred suspension of **intermediate 5** (0.3 g, 1.01 mmol) and pyrimidine-5-boronic acid (0.25 g,
2.02 mmol) in 1,4-dioxane (18 mL) and Na₂CO₃ (aqueous sat. soltn.) (4 mL) at room temperature. The mixture was stirred at 150 °C for 15 minutes under microwave irradiation. The mixture was diluted with water and extracted with DCM. The organic layer was separated, dried (Na₂SO₄), filtered and the solvents evaporated *in vacuo.* The crude product was purified by flash column chromatography (silica gel; 7 M solution of ammonia in methanol in DCM 0/100 to 3/97). The desired fractions were collected and concentrated *in vacuo* to yield **intermediate 6** (0.26 g, 87% yield) as an off-white solid.

### Example A7

### Preparation of intermediate 7: rac-1,3-dimethyl-3-(3-pyrimidin-5-yl-phenyl)-5-thioxopiperazine-2-one

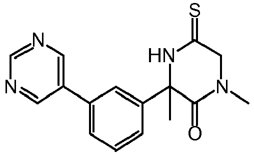

Lawesson's reagent (0.27 g, 0.66 mmol) was added to a stirred solution of **intermediate 6** (0.26 g, 0.60 mmol) and pyridine (0.053 mL, 0.66 mmol) in toluene (9 mL) at room temperature. The mixture was stirred at 90 °C for 18 hours. The solvent was evaporated *in vacuo* and the residue was purified by flash column chromatography (silica gel; 7 M solution of ammonia in methanol in DCM 0/100 to 6/94). The desired fractions were collected and concentrated *in vacuo* to yield **intermediate 7** (0.17 g, 91% yield) as a white solid.

### Example A8

### Preparation of intermediate 8: rac-3-(3-bromo-phenyl)-5-methoxy-1,3-dimethyl-3,6-dihydro-1H-pyrazin-2-one

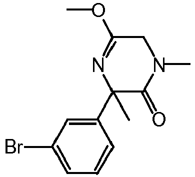

Trimethyloxonium tetrafluoroborate (0.87 g, 5.89 mmol) was added to a stirred solution of **intermediate 5** (0.5 g, 1.68 mmol) in DCM (10 mL) and the mixture was stirred at room temperature for 60 hours. Then the mixture was cooled down to 0 °C, diluted with ice cold NaHCO₃ (aqueous sat. soltn.) and extracted with DCM. The organic layer was separated, dried (Na₂SO₄), filtered and the solvents evaporated *in vacuo* to yield **intermediate 8** (0.51 g, 71 % yield) that was used in the next step without further purification.

### Example A9

### Preparation of intermediate 9: rac-5-amino-3-(3-bromo-phenyl)-1,3-dimethyl-3,6-dihydro-1H-pyrazin-2-one

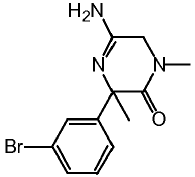

### Method A

Ammonium chloride (0.47 g, 8.77 mmol) was added to a stirred solution of **intermediate 8** (0.45 g, 1.46 mmol) in MeOH (15 mL) in a sealed tube and under nitrogen at room temperature. The mixture was stirred at 85 °C for 18 hours. The solvent was removed *in vacuo* and the residue was diluted with Na₂CO₃ (aqueous sat. soltn.) and extracted with DCM. The organic layer was separated, dried (Na₂SO₄), filtered and the solvents evaporated *in vacuo.* The crude product was purified by ion exchange chromatography using an ISOLUTE^{®} SCX2 cartridge (eluting first with MeOH and then with 7 M solution of ammonia in methanol). The desired fractions eluted with 7 M solution of ammonia in methanol were collected and concentrated *in vacuo* to yield **intermediate 9** (0.16 g, 24% yield) as a brownish oil.

### Method B

32% aqueous ammonia solution (15 mL) was added to **intermediate 10**(0.48 g, 1.53 mmol) and the mixture was stirred in a sealed tube at 50 °C for 18 hours. After cooling to room temperature the mixture was diluted with water and extracted with DCM. The organic layer was separated, dried (Na₂SO₄), filtered and the solvent evaporated *in vacuo* to yield **intermediate 9** (0.45 g, quant. yield) that was used in the next step without further purification.

### Example A10

### Preparation of intermediate 10: rac-3-(3-bromo-phenxl)-1,3-dimethyl-5-thioxopiperazine-2-one

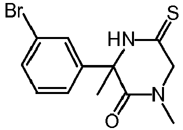

Lawesson's reagent (1.63 g, 4.04 mmol) was added to a stirred solution of **intermediate 5** (1.04 g, 3.36 mmol) and pyridine (0.30 mL, 3.70 mmol) in toluene (33 mL) at room temperature. The mixture was stirred at 90 °C for 18 hours. The solvent was evaporated *in vacuo* and the residue was purified by flash column chromatography (silica gel; MeOH in DCM 0/100 to 4/96). The desired fractions were collected and concentrated *in vacuo* to yield **intermediate 10** (0.5 g, 47% yield) as a colourless oil.

### Example A11

### Preparation of intermediate 11: rac-2-amino-2-(3-nitro-phenyl)-propionitrile

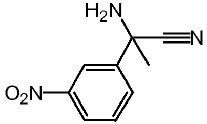

**Intermediate 11** was synthesized following the same approach described in the Example A1. Starting from 1-(3-nitro-phenyl)-ethanone (10 g, 60.55 mmol) **intermediate 11** was obtained as a yellow solid (10.2 g, 88% yield).

### Example A12

### Preparation of intermediate 12: rac-2-amino-2-(3-nitro-phenyl)-propionic acid

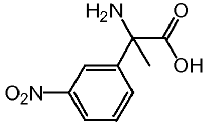

**Intermediate 11** (10.2 g, 53.07 mmol) was added to a 6 N HCl solution (79 mL) at room temperature. The mixture was stirred at reflux for 24 hours. After cooling, water (300 mL) and AcOEt (300 mL) were added. The aqueous layer was separated, partially evaporated *in vacuo* and neutralized by adding a 25% NaOH aqueous solution. The mixture was cooled in an ice-water bath and the precipitate was filtered off, washed with cold water followed by Et₂O and dried *in vacuo* to yield **intermediate 12** (7 g, 63% yield) as a white solid.

### Example A13

### Preparation of intermediate 13: rac-2-amino-2-(3-nitro-phenyl)-propionic acid methyl ester

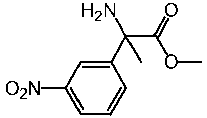

**Intermediate 13** was synthesized following the same approach described in the Example A3. Starting from **intermediate 12** (6 g, 28.55 mmol) **intermediate 13** was obtained as a colourless oil (4 g, 63% yield).

### Example A14

### Preparation of intermediate 14: 2-(2-chloro-acetylamino)-2-(3-nitro-phenxl)-propionic acid methyl ester

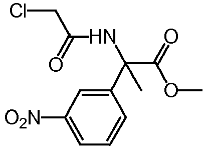

**Intermediate 14** was synthesized following the same approach described in the Example A4. Starting from **intermediate 13** (1.65 g, 7.36 mmol) **intermediate 14** was obtained (2.2 g, 99% yield).

### Example A15

### Preparation of intermediate 15: rac-1,3-dimethyl-3-(3-nitro-phenyl)-piperazine-2,5-dione

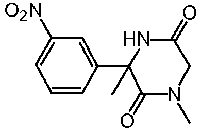

**Intermediate 15** was synthesized following the same approach described in the Example A5. Starting from **intermediate 14** (2.2 g, 7.32 mmol) **intermediate 15** was obtained (1.92 g, quant. yield).

### Example A16

### Preparation of intermediate 16: rac-1,3-dimethyl-3-(3-nitro-phenyl)-5-thioxo-piperazine-2-one

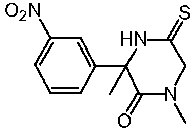

**Intermediate 16** was synthesized following the same approach described in the Example A10. Starting from **intermediate 15** (1.92 g, 7 mmol) **intermediate 16** was obtained as a colourless oil (0.315 g, 16% yield).

### Example A17

### Preparation of intermediate 17: rac-5-amino-1,3-dimethyl-3-(3-nitro-phenyl)-3,6-dihydro-1H-pyrazin-2-one

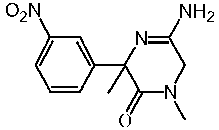

32% aqueous ammonia solution (3 mL) was added to a mixture of **intermediate 16** (0.315 g, 1.13 mmol) in 7 M solution of ammonia in methanol (3 mL) and the mixture was stirred in a sealed tube at 67 °C for 4 hours. After cooling to room temperature the mixture was diluted with water and extracted with DCM. The organic layer was separated, dried (Na₂SO₄), filtered and the solvent evaporated *in vacuo.* The residue was purified by flash column chromatography (silica gel; MeOH in DCM 1/99 to 7/93). The desired fractions were collected and concentrated *in vacuo.* The residue was purified again by flash column chromatography (silica gel; 7 M solution of ammonia in methanol in DCM 7/93 to 10/90). The desired fractions were collected and concentrated *in vacuo* to yield **intermediate 17** (0.11 g, 37% yield).

### Example A18

### Preparation of intermediate 18: rac-5-amino-3-(3-amino-phenyl)-1,3-dimethyl-3,6-dihydro-1H-pyrazin-2-one

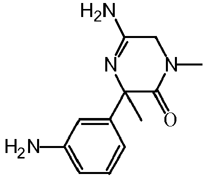

A solution of **intermediate 17** (0.46 g, 1.75 mmol) in EtOH (20 mL) and AcOEt (10 mL) was hydrogenated in a H-Cube reactor (1 mL/min, 30 mm Pd/C 5% cartridge, full H₂ mode, room temperature, 1 cycle). Then, the solvent was evaporated *in vacuo* to yield **intermediate 18** (0.41 g, quant. yield) as a white solid.

### Example A19

### Preparation of intermediate 19: 1-(5-bromo-2,4-difluoro-phenyl)-ethanone

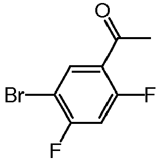

A mixture of AlCl₃ (200 g, 1515.1 mmol) in 1-bromo-2,4-difluoro-benzene (120 g, 621.79 mmol) was stirred at 60 °C for 10 minutes. Then, acetyl chloride (73 g, 929.9 mmol) was added dropwise over 4 hours and the mixture stirred at 95 °C for 6 hours. The mixture was cooled at - 10 °C and ice (300 g) was added over 1 hour. Then, AcOEt was added (500 mL) and the separated organic layer was washed with water, dried (Na₂SO₄), filtered and concentrated *in vacuo.* The residue was purified by flash column chromatography (silica gel; AcOEt in heptane 1/50). The desired fractions were collected and concentrated *in vacuo* to yield **intermediate 19** (60 g, 41 % yield).

### Example A20

### Preparation of intermediate 20: rac-2-amino-2-(5-bromo-2,4-difluoro-phenyl)-propionitrile

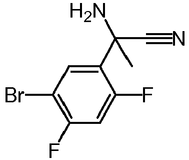

**Intermediate 20** was synthesized following the same approach described in the Example A1. Starting from **intermediate 19** (60 g, 255.31 mmol) **intermediate 20** was obtained (31 g, 47% yield).

### Example A21

### Preparation of intermediate 21 rac-2-amino-2-(5-bromo-2,4-difluoro-phenxl)-propionic acid

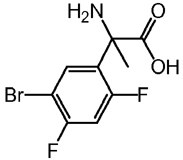

A mixture of **intermediate 20** (28 g, 107.65 mmol) and 6N HCl (300 mL) in AcOH (300 mL) was heated to reflux for 72 hours. After cooling to room temperature, the solvents were evaporated *in vacuo.* AcOEt (400 mL) and water (300 mL) were added. The separated aqueous layer was washed with AcOEt (200 mL). The aqueous layer was separated and adjust to pH = 7. Then, AcOEt (250 mL) was added. The organic layer was separated, dried (Na₂SO₄), filtered and the solvents evaporated *in vacuo* to yield **intermediate 21** (22 g, 72% yield).

### Example A22

### Preparation of intermediate 22: rac-2-amino-2-(5-bromo-2,4-difluoro-phenyl)-propionic acid methyl ester

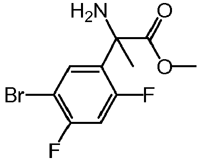

A mixture of **intermediate 21** (22 g, 78.55 mmol) in 4N HCl in MeOH (400 mL) was heated to reflux for 72 hours. After cooling to room temperature, the solvents were evaporated *in vacuo.* AcOEt (400 mL) and water (300 mL) were added. The separated aqueous layer was washed with AcOEt (200 mL). The aqueous layer was separated and adjust to pH = 7. Then, AcOEt (250 mL) was added. The organic layer was separated, dried (Na₂SO₄), filtered and the solvents evaporated *in vacuo* to yield **intermediate 22** (20 g, 87% yield).

### Example A23

### Preparation of intermediate 23: rac-2-(5-bromo-2,4-difluoro-phenyl)-2-(2-chloroacetylamino)-propionic acid methyl ester

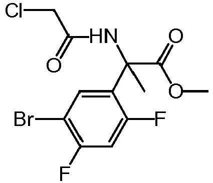

**Intermediate 23** was synthesized following the same approach described in the Example A4. Starting from **intermediate 22** (4 g, 13.60 mmol) **intermediate 23** was obtained (5 g, 99% yield).

### Example A24

### Preparation of intermediate 24: rac-2-(5-bromo-2,4-difluoro-phenyl)-2-(2-ethylaminoacetylamino)-propionic acid methyl ester

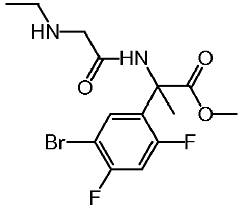

Ethylamine 2 M in THF (4.05 mL, 8.1 mmol) was added to a stirred solution of **intermediate 23** (1 g, 2.7 mmol) in EtOH (12 mL) in a sealed tube at room temperature. Then, the mixture was stirred at 70 °C for 3 hours. The solvent was evaporated *in vacuo* to yield **intermediate 24** (0.55 g, 54% yield) that was used in the next step without further purification.

### Example A25

### Preparation of intermediate 25: rac-3-(5-bromo-2,4-difluoro-phenyl)-1-ethyl-3-methyl-piperazine-2,5-dione

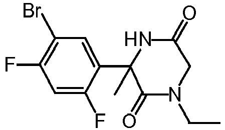

AcOH (0.5 mL) was added to a stirred solution of **intermediate 24** (0.55 g, 1.45 mmol) in EtOH (25 mL) in a sealed tube at room temperature. The mixture was stirred at 95 °C for 16 hours. Then, the mixture was diluted with Na₂CO₃ (aqueous sat. soltn.) and extracted with DCM. The organic layer was separated, dried (Na₂SO₄), filtered and the solvents evaporated *in vacuo.* The crude product was purified by flash column chromatography (silica gel; 7 M solution of ammonia in methanol in DCM 0/100 to 2/98). The desired fractions were collected and concentrated *in vacuo* to yield **intermediate 25** (0.33 g, 66% yield).

### Example A26

### Preparation of intermediate 26: rac-3-[2,4-difluoro-5-(5-methoxy-pyridin-3-yl)-phenyl]-hethyl-3-methyl-piperazine-2,5-dione

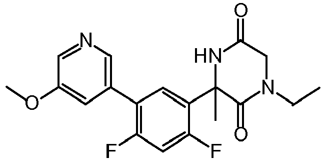

Tetrakis(triphenylphosphine)palladium (0) (0.022 g, 0.019mmol) was added to a stirred suspension of **intermediate 25** (0.33 g, 0.95 mmol) and 3-methoxy-5-pyridineboronic acid (0.19 g, 1.24 mmol) in 1,4-dioxane (12 mL) and Na₂CO₃ (aqueous sat. soltn.) (4 mL) at room temperature. The mixture was stirred at 150 °C for 15 minutes under microwave irradiation. The mixture was diluted with NaHCO₃ (aqueous sat. soltn.) and extracted with DCM. The organic layer was separated, dried (Na₂SO₄), filtered and the solvents evaporated *in vacuo.* The crude product was purified by flash column chromatography (silica gel; MeOH in DCM 0/100 to 11/89). The desired fractions were collected and concentrated *in vacuo* to yield **intermediate 26** (0.26 g, 73% yield) as a colourless oil.

### Example A27

### Preparation of intermediate 27: rac-3-[2,4-difluoro-5-(5-methoxy-pyridin-3-yl)-phenyl]-1-ethyl-3-methyl-5-thioxo-piperazin-2-one

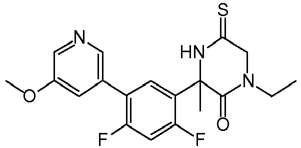

Lawesson's reagent (0.23 g, 0.57 mmol) was added to a stirred solution of **intermediate 26** (0.26 g, 0.47 mmol) and pyridine (0.046 mL, 0.57 mmol) in toluene (9 mL) at room temperature. The mixture was stirred at 90 °C for 18 hours. Then, more Lawesson's reagent (0.23 g, 0.57 mmol) was added and the resulting mixture was heated at 85 °C for 8 hours. Then, more Lawesson's reagent (0.30 g, 0.75 mmol) was added and the resulting mixture was heated at 85 °C for 16 hours. The mixture was diluted with Na₂CO₃ (aqueous sat. soltn.) and extracted with AcOEt. The organic layer was separated, dried (Na₂SO₄), filtered and the solvents evaporated *in vacuo.* The crude product was purified by flash column chromatography (silica gel; MeOH in DCM 0/100 to 6/94). The desired fractions were collected and concentrated *in vacuo* to yield **intermediate 27** (0.14 g, 76% yield).

### B. Preparation of the final compounds

### Example B 1

### Preparation of compound 1: rac-5-amino-1,3-dimethyl-3-(3-pyrimidin-5-yl-phenyl)-3,6-dihydro-1H-pyrazin-2-one

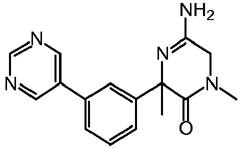

32% aqueous ammonia solution (2 mL) was added **intermediate 7** (0.17 g, 0.54 mmol) and the mixture was stirred in a sealed tube at 65 °C for 2 hours and then at 70 °C for 6 hours. After cooling to room temperature the mixture was diluted with water and extracted with DCM. The organic layer was separated, dried (Na₂SO₄), filtered and the solvent evaporated *in vacuo.* The crude product was purified by flash column chromatography (silica gel; 7 M solution of ammonia in methanol in DCM 0/100 to 6/94). The desired fractions were collected and concentrated *in vacuo* to yield **compound 1** (0.09 g, 56% yield) as a white solid.

### Example B2

### Preparation of compound 2: rac-5-amino-3-[3-(5-methoxy-pyridin-3-yl)-phenyl]-1,3-dimethyl-3,6-dihydro-1H-pyrazin-2-one

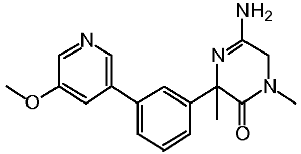

EtOH (3 mL) was added to a mixture of **intermediate 9** (0.16 g, 0.35 mmol), trans-bisdicyclohexylamine)palladium diacetate [DAPCy, CAS 628339-96-8] (0.021 g, 0.035 mmol), potassium phosphate (0.22 g, 1.05 mmol) and 3-methoxy-5-pyridine-boronic acid pinacol ester (0.12 g, 0.53 mmol). The mixture was stirred at 80 °C for 48 hours. After cooling the mixture was diluted with water and Na₂CO₃ (aqueous sat. soltn.) and extracted with DCM. The organic layer was separated, dried (Na₂SO₄), filtered and the solvents evaporated *in vacuo.* The crude product was purified by flash column chromatography (silica gel; 7 M solution of ammonia in methanol in DCM 0/100 to 7/93). The desired fractions were collected and concentrated *in vacuo* and the crude product was purified again by flash column chromatography (silica gel; 7 M solution of ammonia in methanol in DCM 0/100 to 7/93). The desired fractions were collected and concentrated *in vacuo* to yield **compound 2** (0.013 g, 11% yield).

### Example B3

### Preparation of compound 3: rac-5-chloro-pyridine-2-carboxylic acid [3-(6-amino-2,4-dimethyl-3-oxo-2,3,4,5-tetrahydro-pyrazin-2-yl)-phenyl]-amide

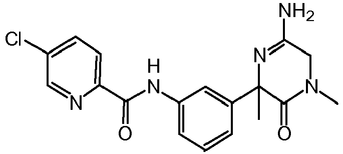

### Method A

Trans-1,2-diaminocyclohexane (0.002 g, 0.018 mmol) was added to a stirred suspension of **intermediate 9** (0.052 g, 0.176 mmol), copper(I) iodide (0.002 g, 0.009 mmol), 5-chloro-2-pyridinecarboxamide (0.028 g, 0.176 mmol) and potassium phosphate tribasic (0.075 g, 0.351 mmol) in DMF (1 mL) in a sealed tube and under nitrogen at room temperature. The mixture was stirred at 180 °C for 140 minutes under microwave irradiation. The mixture was diluted with NH₄Cl (aqueous sat. soltn.) and extracted with DCM. The organic layer was separated, dried (MgSO₄), filtered and the solvents evaporated *in vacuo.* The crude product was purified by flash column chromatography (silica gel; 7 M solution of ammonia in methanol in DCM 0/100 to 1/99). The desired fractions were collected and concentrated *in vacuo* to yield **compound 3**(0.004 g, 6% yield).

### Method B

5-Chloro-2-pyridinecarboxylic acid (0.234 g, 1.485 mmol) was added to a suspension of **intermediate 18** (0.3 g, 1.292 mmol) in DCM (13 mL) at room temperature. Then, *N,N-*dimethylaniline (0.21 mL, 1.679 mmol) was added and after stirring at room temperature for 5 minutes HATU (0.54 g, 1.421 mmol) was added. The mixture was stirred at room temperature for 16 hours. The mixture was diluted with water and extracted with DCM. The organic layer was separated, dried (Na₂SO₄), filtered and the solvents evaporated *in vacuo.* The crude product was purified by flash column chromatography (silica gel; methanol in DCM 0/100 to 10/90). The desired fractions were collected and concentrated *in vacuo* to yield **compound 3** (0.294 g, 61 % yield).

### Example B4

### Preparation of compound 4: (S*)-5-chloro-pyridine-2-carboxylic acid [3-(6-amino-2,4-dimethyl-3-oxo-2,3,4,5-tetrahydro-pyrazin-2-yl)-phenyl]-amide and compound 5 (R*)-5-chloro-pyridine-2-carboxylic acid [3-(6-amino-2,4-dimethyl-3-oxo-2,3,4,5-tetrahydro-pyrazin-2-yl)-phenyl]-amide

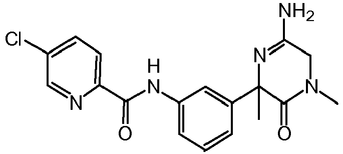

A sample of **compound 3** (294 mg) was separated into the corresponding enantiomers by preparative SFC on Chiralcel^{®} OD-H (5 µm 250 x 20 mm), mobile phase (0.3% isopropyl-amine, 60% CO₂, 40% mixture of EtOH/iPrOH 50/50 v/v), yielding **compound 4** (0.11 g) and **compound 5** (0.15 g). This last derivative was purified again by flash column chromatography (silica gel; 0.5% NH₄OH, 95% DCM, 5% EtOH) to yield pure **compound 5** (0.09 g).

### Example B5

### Preparation of compound 6: rac-5-methyl-pyrazine-2-carboxylic acid [3-(6-amino-2,4-dimethyl-3-oxo-2,3,4,5-tetrahydro-pyrazin-2-yl)-phenyl]-amide

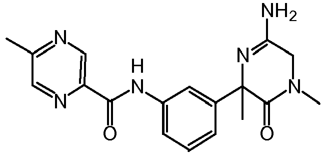

5-Methylpyrazine-2-carboxylic acid (0.014 g, 0.104 mmol) was added to a suspension of **intermediate 18** (0.021 g, 0.09 mmol) in DCM (1.5 mL) at room temperature. Then, pyridine (0.01 mL, 0.118 mmol) was added and after stirring at room temperature for 5 minutes HATU (0.038 g, 0.099 mmol) was added. The mixture was stirred at room temperature for 16 hours. The mixture was diluted with water and extracted with DCM. The organic layer was separated, dried (Na₂SO₄), filtered and the solvents evaporated *in vacuo.* The crude product was purified by flash column chromatography (silica gel; MeOH in DCM 0/100 to 10/90). The desired fractions were collected and concentrated *in vacuo.* The residue was purified again by flash column chromatography (silica gel; solid injection; 7 M solution of ammonia in methanol in DCM 0/100 to 2/98). The desired fractions were collected and concentrated *in vacuo* to yield **compound 6** (0.009 g, 28% yield).

### Example B6

### Preparation of compound 9: rac-5-amino-3-[2,4-difluoro-5-(5-methoxy-pyridin-3-yl)-phenyl]-hethyl-3-methyl-3,6-dihydro-1H-pyrazin-2-one

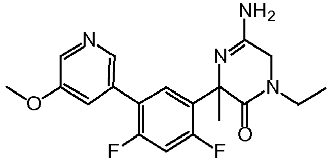

32% aqueous ammonia solution (8 mL) was added to a solution of **intermediate 27** (0.14 g, 0.36 mmol) in 7 M solution of ammonia in methanol (4 mL) and the mixture was stirred in a sealed tube at 65 °C for 3 hours. After cooling to room temperature the mixture was diluted with water and extracted with DCM. The organic layer was separated, dried (Na₂SO₄), filtered and the solvent evaporated *in vacuo* to yield compound 9 (0.12 g, 90% yield) as a white solid.

**Table 1**

| | | | | | | |
|---|---|---|---|---|---|---|
| 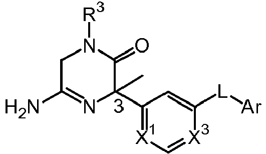 | | | | | | |

| **Co. No.** | **Ex. No.** | **---R³** | **X¹** | **X³** | **---L-Ar** | **C₃-stereochemistry** |
|---|---|---|---|---|---|---|
| **1** | B1 | ---Me | CH | CH | 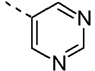 | RS |
| **2** | B2 | ---Me | CH | CH | 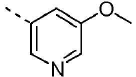 | RS |
| **3** | B3 | ---Me | CH | CH | 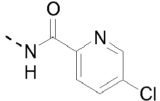 | RS |
| **4** | B4 | ---Me | CH | CH | 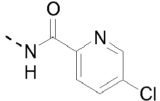 | S* |
| **5** | B4 | ---Me | CH | CH | 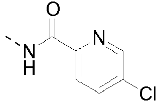 | R* |
| **6** | B5 | ---Me | CH | CH | 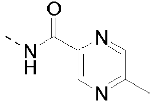 | RS |
| **7** | B5 | ---Me | CH | CH | 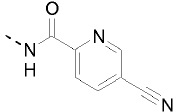 | RS |
| **8** | B5 | ---Me | CH | CH | 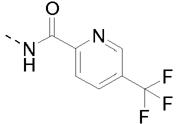 | RS |
| **9** | B6 | ---Et | CF | CF | 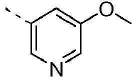 | RS |

### C. Analytical Part

### LCMS

For (LC)MS-characterization of the compounds of the present invention, the following methods were used.

### General procedure A:

The UPLC (Ultra Performance Liquid Chromatography) measurement was performed using an Acquity UPLC (Waters) system comprising a sampler organizer, a binary pump with degasser, a four column's oven, a diode-array detector (DAD) and a column as specified in the respective methods. The MS detector was configured with an ESCI dual ionization source (electrospray combined with atmospheric pressure chemical ionization). Nitrogen was used as the nebulizer gas. The source temperature was maintained at 140 °C. Data acquisition was performed with MassLynx-Openlynx software.

### Method 1:

In addition to the general procedure A: Reversed phase UPLC was carried out on a BEH-C18 column (1.7 µm, 2.1 x 50 mm) from Waters, with a flow rate of 1.0 ml/min, at 50°C without split to the MS detector. The gradient conditions used are: 95 % A (0.5 g/l ammonium acetate solution + 5 % acetonitrile), 5 % B (acetonitrile), to 40 % A, 60 % B in 3.8 minutes, to 5 % A, 95 % B in 4.6 minutes, kept till 5.0 minutes. Injection volume 2 µl. Low-resolution mass spectra (single quadrupole, SQD detector) were acquired by scanning from 100 to 1000 in 0.1 seconds using an inter-channel delay of 0.08 second. The capillary needle voltage was 3 kV. The cone voltage was 25 V for positive ionization mode and 30 V for negative ionization mode.

### General procedure B:

The LC measurement was performed using a UPLC (Ultra Performance Liquid Chromatography) Acquity (Waters) system comprising a binary pump with degasser, an autosampler, a diode-array detector (DAD) and a column as specified in the respective methods below, the column is hold at a temperature of 40°C. Flow from the column was brought to a MS detector. The MS detector was configured with an electrospray ionization source. The capillary needle voltage was 3 kV and the source temperature was maintained at 130 °C on the Quattro (triple quadrupole mass spectrometer from Waters). Nitrogen was used as the nebulizer gas. Data acquisition was performed with MassLynx-Openlynx software (Waters).

### Method 2:

In addition to the general procedure B: Reversed phase UPLC was carried out on a Waters Acquity BEH (bridged ethylsiloxane/silica hybrid) Phenyl-Hexyl column (1.7 µm, 2.1 x 100 mm) with a flow rate of 0.343 ml/min. Two mobile phases (mobile phase A: 95 % 7 mM ammonium acetate / 5 % acetonitrile; mobile phase B: 100 % acetonitrile) were employed to run a gradient condition from 84.2 % A and 15.8 % B (hold for 0.49 minutes) to 10.5 % A and 89.5 % B in 2.18 minutes, hold for 1.94 min and back to the initial conditions in 0.73 min, hold for 0.73 minutes. An injection volume of 2 ml was used. Cone voltage was 20V for positive and negative ionization mode. Mass spectra were acquired by scanning from 100 to 1000 in 0.2 seconds using an interscan delay of 0.1 seconds.

### Melting Points

Values are either peak values or melt ranges, and are obtained with experimental uncertainties that are commonly associated with this analytical method.

### Mettler FP 81HT / FP90 apparatus (indicated by FP90 in Table 2)

For a number of compounds, melting points were determined in open capillary tubes on a Mettler FP81HT / FP90 apparatus. Melting points were measured with a temperature gradient of 1, 3, 5 or 10 °C/minute. Maximum temperature was 300 °C. The melting point was read from a digital display.

**Table 2: Analytical data - Rₜ means retention time (in minutes), [M+H]⁺ means the protonated mass of the compound, method refers to the method used for (LC)MS.**

| **Co. Nr.** | **Rt** | **[M+H]⁺** | **Method** | **Melting Point** |
|---|---|---|---|---|
| **1** | 0.44 | 296 | 1 | 223.4 °C (FP90) |
| **2** | 0.91 | 325 | 1 | n.d. |
| **3** | 1.22 | 372 | 1 | 148.1 °C (FP90) |
| **4** | 2.06 | 372 | 2 | 201.3 °C (FP90) |
| **5** | 2.06 | 372 | 2 | 218.5 °C (FP90) |
| **6** | 0.69 | 353 | 1 | n.d. |
| **7** | 0.84 | 363 | 1 | n.d. |
| **8** | 1.59 | 406 | 1 | n.d. |
| **9** | 1.14 | 375 | 1 | 81.8 °C (FP90) |

| | | | | |
|---|---|---|---|---|
| n.d. means not determined | | | | |

### SFCMS

### General procedure

The SFC measurement was performed using an Analytical SFC system from Berger instruments (Newark, DE, USA) comprising a FCM-1200 dual pump fluid control module for delivering carbon dioxide (CO2) and modifier, a CTC Analytics automatic liquid sampler, a TCM-20000 thermal control module for column heating from room temperature to 80°C. An Agilent 1100 UV photodiode array detector equipped with a high-pressure flow cell standing up to 400 bars was used. Flow from the column was split to a MS spectrometer. The MS detector was configured with an atmospheric pressure ionization source .The following ionization parameters for the Waters ZQ mass spectrophotometer are: corona: 9µa, source temp: 140°C, cone: 30 V, probe temp 450°C, extractor 3 V, desolvatation gas 400L/hr, cone gas 70 L/hr. Nitrogen was used as the nebulizer gas. Data acquisition was performed with a Waters-Micromass MassLynx-Openlynx data system.

### Method 1:

In addition to the general procedure: The chiral separation in SFC was carried out on Chiralcel^{®} OD DAICEL column (10 µm, 4.6 x 250 mm) at 35°C with a flow rate of 3.0 ml/min. The mobile phase is CO₂, 40% Ethanol/Isopropanol (1/1) (containing 0.3% iPrNH₂) hold 7 min.

**Table 3: Analytical SFC data - Rₜ means retention time (in minutes), [M+H]⁺ means the protonated mass of the compound, method refers to the method used for (SFC)MS analysis of enantiomerically pure compounds.**

| **Co. Nr.** | **Rt** | **[M+H]⁺** | **UV Area %** | **Method** | **Isomer Elution Order** |
|---|---|---|---|---|---|
| **4** | 4.41 | 372 | 100 | 1 | A |
| **5** | 5.47 | 372 | 100 | 1 | B |

### Optical Rotations

Optical rotations were measured on a Perkin-Elmer 341 polarimeter with a sodium lamp and reported as follows: [α]_{λ}^{t°C} (cg/100ml, solvent).

**Table 4: Analytical data - Optical rotation values for enantiomerically pure compounds**

| **Co. Nr.** | **a_{D} (°)** | **Wavelength (nm)** | **Concentration w/v%** | **Solvent** | **Temp. (° C)** |
|---|---|---|---|---|---|
| **4** | +45.3 | 589 | 0.72 | DMF | 20 |
| **5** | -45.7 | 589 | 0.49 | DMF | 20 |

### D. Pharmacological examples

The compounds provided in the present invention are inhibitors of the beta-site APP-cleaving enzyme 1 (BACE1). Inhibition of BACE1, an aspartic protease, is believed to be relevant for treatment of Alzheimer's Disease (AD). The production and accumulation of beta-amyloid peptides (Abeta) from the beta-amyloid precursor protein (APP) is believed to play a key role in the onset and progression of AD. Abeta is produced from the amyloid precursor protein (APP) by sequential cleavage at the N- and C-termini of the Abeta domain by beta-secretase and gamma-secretase, respectively.

Compounds of Formula (I) are expected to have their effect substantially at BACE1 by virtue of their ability to inhibit the enzymatic activity. Inhibitors were tested using a biochemical Fluorescence Resonance Energy Transfer (FRET) based assay and a cellular αLisa assay in SKNBE2 cells as described below, The results are shown in Tables 5 and 6.

### Biochemical FRET based assay

This assay is a Fluorescence Resonance Energy Transfer Assay (FRET) based assay. The substrate for this assay is an APP derived 13 amino acids peptide that contains the 'Swedish' Lys-Met/Asn-Leu mutation of the amyloid precursor protein (APP) beta-secretase cleavage site. This substrate also contains two fluorophores: (7-methoxycoumarin-4-yl) acetic acid (Mca) is a fluorescent donor with excitation wavelength at 320 nm and emission at 405 nm and 2,4-Dinitrophenyl (Dnp) is a proprietary quencher acceptor. The distance between those two groups has been selected so that upon light excitation, the donor fluorescence energy is significantly quenched by the acceptor, through resonance energy transfer. Upon cleavage by BACE1, the fluorophore Mca is separated from the quenching group Dnp, restoring the full fluorescence yield of the donor. The increase in fluorescence is linearly related to the rate of proteolysis.

Briefly in a 384-well format recombinant BACE1 protein in a final concentration of 1µg/ml is incubated for 120 minutes at room temperature with 10 µm substrate in incubation buffer (40mM Citrate buffer pH 5.0, 0.04% PEG, 4% DMSO) in the absence or presence of compound. Next the amount of proteolysis is directly measured by fluorescence measurement at T=0 and T=120 (excitation at 320 nm and emission at 405 nm). Results are expressed in RFU (relative fluorescence units), as difference between T120 and T0.

A best-fit curve is fitted by a minimum sum of squares method to the plot of %Controlmin versus compound concentration. From this an IC50 value (inhibitory concentration causing 50% inhibition of activity) can be obtained.
LC = Median of the low control values
   = Low control: Reaction without enzyme
HC = Median of the High control values
   = High Control: Reaction with enzyme
%Effect = 100-[(sample-LC) / (HC-LC) *100]
%Control = (sample /HC)*100
%Controlmin = (sample-LC) / (HC-LC) *100

The following exemplified compounds were tested essentially as described above and exhibited the following the activity:

**Table 5**

| **Co. Nr.** | **Biochemical FRET based assay pIC₅₀** |
|---|---|
| **1** | 4.69 |
| **2** | 4.98 |
| **3** | 6.11 |
| **4** | < 4.52 |
| **5** | 6.49 |
| **6** | 5.15 |
| **7** | 6.09 |
| **8** | 5.86 |
| **9** | < 4.52 |

### Cellular αLisa assay in SKNBE2 cells

In two αLisa assays the levels of Abeta total and Abeta 1-42 produced and secreted into the medium of human neuroblastoma SKNBE2 cells are quantified. The assay is based on the human neuroblastoma SKNBE2 expressing the wild type Amyloid Precursor Protein (hAPP695). The compounds are diluted and added to these cells, incubated for 18 hours and then measurements of Abeta 1-42 and Abeta total are taken. Abeta total and Abeta 1-42 are measured by sandwich αLisa. αLisa is a sandwich assay using biotinylated antibody AbN/25 attached to streptavidin coated beads and antibody Ab4G8 or cAb42/26 conjugated acceptor beads for the detection of Abeta total and Abeta 1-42 respectively. In the presence of Abeta total or Abeta 1-42, the beads come into close proximity. The excitation of the donor beads provokes the release of singlet oxygen molecules that trigger a cascade of energy transfer in the acceptor beads, resulting in light emission. Light emission is measured after 1 hour incubation (excitation at 650 nm and emission at 615 nm).

A best-fit curve is fitted by a minimum sum of squares method to the plot of %Controlmin versus compound concentration. From this an IC50 value (inhibitory concentration causing 50% inhibition of activity) can be obtained.
LC = Median of the low control values
   = Low control: cells preincubated without compound, without biotinylated Ab in the αlisa
HC = Median of the High control values
   = High Control: cells preincubated without compound
%Effect = 100-[(sample-LC) / (HC-LC) *100]
%Control = (sample /HC)*100
%Controlmin = (sample-LC) / (HC-LC) *100

The following exemplified compounds were tested essentially as described above and exhibited the following the activity:

**Table 6**

| **Co. Nr.** | **Cellular αlisa assay in SKNBE2 cells Aß42 pIC₅₀** | **Cellular αlisa assay in SKNBE2 cells Aßtotal pIC₅₀** |
|---|---|---|
| **1** | 5.35 | 5.41 |
| **2** | 5.78 | 5.82 |
| **3** | 7.19 | 7.56 |
| **4** | < 5 | < 5 |
| **5** | 7.44 | 7.43 |
| **6** | 5.90 | 5.94 |
| **7** | 6.73 | 6.82 |
| **8** | 7.17 | 7.10 |
| **9** | < 5 | 5.04 |

## Claims

1. A compound of Formula (I) 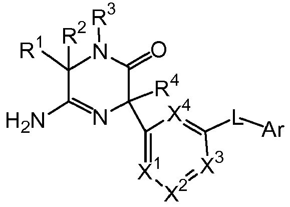 or a stereoisomeric form thereof, wherein
R¹, R² are hydrogen;
R³, R⁴ are independently methyl or ethyl;
X¹ and X³ are CH or CF;
X² and X⁴ are CH;
L is a bond or -N(R⁶)CO- wherein R⁶ is hydrogen;
Ar is heteroaryl;
heteroaryl is selected from the group consisting of pyridyl, pyrimidinyl and pyrazyl, each optionally substituted with chloro, cyano, methyl, methoxy or trifluoromethyl.

2. The compound according to claim 1 wherein
R¹, R² are hydrogen;
R³, R⁴ are methyl;
X¹, X², X³, X⁴ are CH;
L is -N(R⁶)CO- wherein R⁶ is hydrogen;
Ar is heteroaryl;
heteroaryl is pyridyl substituted with chloro, cyano, methoxy or trifluoromethyl, pyrimidinyl, or pyrazyl substituted with methyl.

3. A pharmaceutical composition comprising a therapeutically effective amount of a compound as defined in claim 1 or 2 and a pharmaceutically acceptable carrier.

4. A process for preparing a pharmaceutical composition as defined in claim 3, **characterized in that** a pharmaceutically acceptable carrier is intimately mixed with a therapeutic ally effective amount of a compound as defined in claim 1 or 2.

5. A compound as defined in claim 1 or 2 for use in the treatment, prevention or prophylaxis of Alzheimer's disease (AD), mild cognitive impairment, senility, dementia, dementia with Lewy bodies, Down's syndrome, dementia associated with stroke, dementia associated with Parkinson's disease or dementia associated with beta-amyloid.

## Patentansprüche

1. Verbindung der Formel (I) 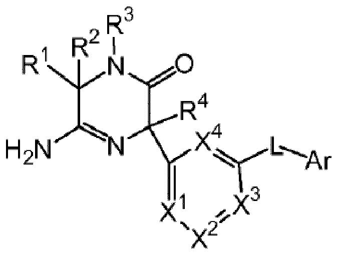 oder stereoisomere Form davon, wobei
R¹, R² für Wasserstoff stehen;
R³, R⁴ unabhängig voneinander für Methyl oder Ethyl stehen;
X¹ und X³ für CH oder CF stehen;
X² und X⁴ für CH stehen;
L für eine Bindung oder -N(R6)CO- steht, wobei R⁶ für Wasserstoff steht;
Ar für Heteroaryl steht;
Heteroaryl aus der aus Pyridyl, Pyrimidinyl und Pyrazyl bestehenden Gruppe ausgewählt ist, wobei diese Reste jeweils gegebenenfalls durch Chlor, Cyano, Methyl, Methoxy oder Trifluormethyl substituiert sind.

2. Verbindung nach Anspruch 1, wobei
R¹, R² für Wasserstoff stehen;
R³, R⁴ für Methyl stehen;
X¹, X², X³, X⁴ für CH stehen;
L für -N(R6)CO- steht, wobei R⁶ für Wasserstoff steht;
Ar für Heteroaryl steht;
Heteroaryl für durch Chlor, Cyano, Methoxy oder Trifluormethyl substituiertes Pyridyl, Pyrimidinyl oder durch Methyl substituiertes Pyrazyl steht.

3. Pharmazeutische Zusammensetzung, umfassend eine therapeutisch wirksame Menge einer wie in Anspruch 1 oder 2 definierten Verbindung und einen pharmazeutisch unbedenklichen Träger.

4. Verfahren zur Herstellung einer wie in Anspruch 3 definierten pharmazeutischen Zusammensetzung, **dadurch gekennzeichnet, dass** man einen pharmazeutisch unbedenklichen Träger innig mit einer therapeutisch wirksamen Menge einer wie in Anspruch 1 oder 2 definierten Verbindung mischt.

5. Wie in Anspruch 1 oder 2 definierte Verbindung zur Verwendung bei der Behandlung, Prävention oder Prophylaxe von Alzheimer-Krankheit (AD), milder kognitiver Störung, Senilität, Demenz, Demenz mit Lewy-Körperchen, Down-Syndrom, mit Schlaganfall assoziierter Demenz, mit Parkinson-Krankheit assoziierter Demenz oder mit beta-Amyloid assoziierter Demenz.

## Revendications

1. Composé de formule (I) 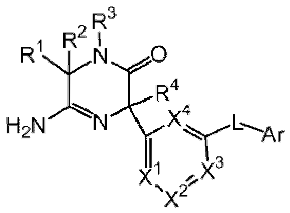 ou une forme stéréoisomère de celui-ci, dans lequel
R¹, R² sont hydrogène ;
R³ R⁴ sont indépendamment méthyle ou éthyle ;
X¹ et X³ sont CH ou CF ;
X² et X⁴ sont CH ;
L est une liaison ou -N (R⁶) CO- où R⁶ est hydrogène ;
Ar est hétéroaryle ;
l'hétéroaryle est choisi dans le groupe constitué de pyridyle, pyrimidinyle et pyrazyle, chacun étant facultativement substitué par chloro, cyano, méthyle, méthoxy ou trifluorométhyle.

2. Composé selon la revendication 1 dans lequel
R¹, R² sont hydrogène ;
R³, R⁴ sont méthyle ;
X¹, X², X³, X⁴ sont CH ;
L est -N (R⁶) CO- où R⁶ est hydrogène ;
Ar est hétéroaryle ;
hétéroaryle étant pyridyle substitué par chloro, cyano, méthoxy ou trifluorométhyle, pyrimidinyle, ou pyrazyle substitué par méthyle.

3. Composition pharmaceutique comprenant une quantité thérapeutiquement efficace d'un composé tel que défini dans la revendication 1 ou 2 et un véhicule pharmaceutiquement acceptable.

4. Procédé pour préparer une composition pharmaceutique tel que défini dans la revendication 3, **caractérisé en ce qu'**un véhicule pharmaceutiquement acceptable est intimement mélangé avec une quantité thérapeutiquement efficace d'un composé tel que défini dans la revendication 1 ou 2.

5. Composé tel que défini dans la revendication 1 ou 2 pour utilisation dans le traitement, la prévention ou la prophylaxie de la maladie d'Alzheimer (MA), un trouble cognitif léger, la sénilité, la démence, la démence à corps de Lewy, le syndrome de Down, la démence associée à un accident vasculaire cérébral, la démence associée à la maladie de Parkinson ou la démence associée au bêta-amyloïde.
